# EUROPEAN PATENT APPLICATION

(11) **EP 2 862 513 A2**
(43) Date of publication of application: **22.04.2015**
(21) Application number: 14189485.7
(22) Date of filing: 20.10.2014
(51) Int. Cl.: A61B 5/055, G01R 33/34, A61B 6/04, A61B 6/00, G01R 33/38

(54) **MR imaging with RF coil integrated into patient engaging component**

(30) Priority: 18.10.2013 US 201314057622
(71) Applicant: Imris Inc., Winnipeg, Manitoba R3T 1N5 (CA)
(72) Inventor: Petropoulos, Labros, Minnetonka, MN 55343 (US); Heinz, Eric S., Minnetrista, MN 55331 (US); Sarafa, John, Minnetonka, MN 55343 (US); Vanney, Guy, Blaine, MN 55434 (US)
(74) Representative: Jostarndt, Hans-Dieter

(57) **Abstract**

In MR imaging of a body part in the magnetic field of a magnet, an RF signal is applied in a transmit stage to the subject to be imaged such that the subject generates an MR signal in response to the magnetic field and the RF signal applied with the MR signal being acquired in a receive stage using an RF coil where the RF coil is an integral element defined within a supporting element such as a pillow or U-shaped head support carrying the body part or within an article worn or carried by the body part such as a cast, brace, brassiere or vest.

## Description

This invention relates to an RF coil assembly for MR imaging.

### BACKGROUND OF THE INVENTION

As is well known, MR imaging uses an RF receive coil to receive the signals emitted by the subject under test in response to excitation of a selected volume of the subject which is generated by a RF transmit coil, such as the built in body coil. Thus the Gradient coils generate controlled variations in the main magnetic field (B0) magnetic field to produce selected spatial excitation volume and the signal emitted by that selected volume is picked up by the receive coil arrangement and transmitted to a signal processing system.

The receive coil arrangement can comprises a single coil loop or element or it can include a series of loops arranged in a pattern around the part of the subject to be imaged.

MR systems provide a built in body coil in the magnet construction and this can operate as both the transmit coil and the receive coil.

However in some cases the body coil does not provide an image of sufficient quality to meet the requirements and hence local coils must be used. These can be simple coil loops or more complex volume coils which are configured to at least partially or completely surround the region of interest of the subject so as to receive the MR signal and include a plurality of connected conductors.

The development of intraoperative MRI as described in US patent 5735278 (Hoult) created a need to develop supporting accessories which both streamline the surgical workflow and enable the acquisition of diagnostic quality intraoperative images. Examples of supporting accessories are the RF coils used to optimize the quality of the MRI image. Existing technologies require the use of rigid or flexible RF coils, which were primarily designed for use in the diagnostic imaging environment. In the surgical environment, these coils are difficult to position close to the anatomy in a way that preserves the sterile field.

In many surgical procedures, the target anatomy is held in place with some type of patient positioning device. These patient positioning devices were not designed with intraoperative imaging in mind, so they are not optimal for use in an intraoperative imaging environment. As a result, the RF coils must be placed a significant distance away from the anatomy, which results in degradation of the signal to noise ratio. Degradation of image homogeneity takes place as well, since the gap between the two phase array coils is significant.

When the existing coils are in place, they can obstruct the working space for the surgeon. As a result, additional OR time must be taken to place and remove these coils from the patient when intraoperative imaging takes place.

Due to the nature of intraoperative imaging, the cable that connects the coil to the MRI system is long, heavy, and cumbersome to handle. Depending upon the position of this cable relative to the bore, RF heating may occur, which creates a safety concern.

Rigid and flexible RF coils are also very expensive, and the wear and tear these devices see in the operating room environment result in expensive repairs and replacements for the customer. Some current volume coils consist of coil loops, phased array, birdcage, TEM, all of which could be single frequency or dual frequency coils. These require matching networks, preamplifiers, decoupling networks, cables and connectors.

There are a number of challenges with the current standard volume coil designs:
a) The number of channels is limited to the number of receivers in the system.
b) A large diameter cable bundle, such as an eighteen channel phased array coil which require 18 channel cables, containing 18 coaxial cables and at least 25 control wires, would be much too large to enable construction of the conventional cable trap in the cable.
c) It is difficult to build because the electrical components, such as the circuit board baluns and preamps, are complicated and time consuming to assemble by a skilled and experienced technician. These components require significant effort during design and construction to produce high quality images and to reduce the crosstalk between components.
d) The required mechanical components, such as the long cables, cable traps, and connector interface also increase the overall size and weight of the coil.
e) The large size and weight of the coils increases complexity of workflow for customer and complexity of the workflow design.
f) Long cables are heavy and cumbersome to position.
g) There are patient positioning and surgical access issues due to the inflexibility of the current design and the ever-changing surgical requirements and surgeon's preferences.
h) Coil cables have the possibility of patient burns resulting from skin-to-cable contact, resulting in increased space between cables, magnet bore and patient. This provides less in less patient space for nursing staff to properly position the patient before scanning.
i) In an inter-operative suite, there are safety issues related to OR staff forgetting to unplug the coils and increased OR workflow due to the additional patient safety checkpoint. Normally, each individual loop or loops of the MRI receive coil arrangement are connected to a single receiver of the signal processing system via preamplifier and other components with a cable.

Such receive coil arrangements can therefore use the so called "built in body coil" carried on the magnet as receive coil which is connected by cable to the signal processing system. In this case the so called "built in body coil" is also used as transmit coil

Such receive coil arrangements can therefore comprise a single loop which is connected by a single wire to a single channel of the signal processing system. In this case the system can use the so called "built in body coil" carried on the magnet as transmit coil. This signal loop receive coil then supplies the received signal collected around the subject, typically a lying patient, and communicates it to the single channel for processing using conventional systems well known to persons in this art.

Such receive coil arrangements can therefore comprise a multiple loop arrangement including a so-called "phased array" of loops each of which is connected by a respective wire to a separate one of a plurality of channels of the signal processing system.

In this case the system typically uses a portable coil assembly arranged to wrap around the body part of the patient but each loop must have its own set of processing components and its own wire connecting the signal to the separate channel for processing.

However in recent developments not yet widely adopted, the "built in body coil" carried on the magnet as the receive coil arrangement is separated into individual loop components for supplying a separate signal to the separate channels.

It is well known that there are parallel imaging techniques to reduce the time necessary to obtain a complete scan of the part of the patient by using the signals from the separate channels to carry out various calculations and extrapolations, thus avoiding the necessity to obtain image results at each location in the image space or in K-space. Some of these parallel imaging techniques are known as SMASH and SENSE and GRAPPA.

To obtain better images, the preamplifiers are located as close to the coil elements as possible. Although the size of MR preamplifier is greatly reduced recently, it still takes much space of overall array coil. In addition the area of coil enclosure at preamplifier must be rigid.

The coil cable, as is well known, consists of multi-coaxial cable and signal control wires and outer shield. Common mode current or shield current will be generated on outer surface of the shield during transmit phase by the high RF field generated by the transmit coil. To prevent the patient from being overheated dangerously by shield current, cable traps are required for the coil cable assembly. Longer cable with more cable traps is required for the clinic applications, such as intra-operative MR imaging on a moving magnetic system.

Another issue which arises with the complex coil arrangements set forth above is that of increased time required to deploy the RF coli construction on the patient prior to the imaging taking place.

The ultimate goal is to achieve the best signal to noise ratio and image homogeneity as possible. The proximity of the RF coil to the anatomy is a primary factor in achieving this goal. Unfortunately up to this point, the only possibility is to incorporate sophisticated mounting mechanisms for the coils to be near the anatomy.

### SUMMARY OF THE INVENTION

It is an object of the present invention to provide an RF coil arrangement which enables the coil to be more easily deployed for the patient.

According to a first aspect of the invention there is provided a method for MR imaging of a subject comprising
generating a variable magnetic field to be applied to a body part to be imaged of the subject; supporting the body part of the subject in the magnetic field by engaging the body part with a supporting element;
transmitting an RF signal in a transmit stage to be applied to the subject to be imaged such that the subject generates an MR signal in response to the magnetic field and the RF signal applied; acquiring the MR signal in a receive stage using an RF coil;
and processing the MR signal for generating an image;
wherein the RF coil is an integral element with the supporting element.

In one example, the RF coil is embedded within the supporting element. In this case the supporting element can be a resilient material on which the body part to be imaged rests. This can be any required support such as typically a horse shoe shaped head support. However other support elements such as bolsters that support the pelvis for hip/pelvic imaging, in a knee brace, in an ankle brace, in a cast, in a cervical spine collar, in a prone positioning head pillow, or in a vacuum "beanbag" positioner.

Preferably the subject is carried into the magnet on a support table and the supporting element is removable from the table and when removed carries the RF coil as an integral element therewith.

In one example, the RF coil is integrated with the support element by insert molding of the coil circuitry thereof directly into a resilient material forming the structure of the support element. This arrangement is used with supports which are wholly or primarily of a resilient material such as foam or gel.

In another arrangement the RF coil is integrated with the support element by encapsulating a coil circuitry thereof in a rigid component upon which a resilient material is over-molded or adhered to with adhesive. This can be used where the support is structural and wholly or primarily formed of rigid materials such as a head clamp.

In yet another arrangement, the RF coil is integrated with the support element by extruding a resilient material with a strip of coil circuitry thereof which is fed into the extruded resilient material and the extruded material is cut into lengths. In this arrangement, the lumped element components such capacitors, inductors and diodes are encapsulated into a safe plastic housing that is not affected by the extruding process. All of the components of the coil including the copper traces and the above elements in the housing are introduced into the extrusion process and formed together. When the extruded product is cut into lengths there are provided, at both ends, parts of the coil elements extending that can be connected together with soldering or mechanical means. The coil elements in the separate extruded portions can be connected to form a loop, a set of loops, or combinations of loops and butterflies or any other designed two dimensional or three dimensional shaped RF coil structure.

According to a second aspect of the invention there is provided a method for obtaining an MR image of a subject in an MR imaging system comprising
generating a variable magnetic field to be applied to a body part to be imaged of the subject; supporting the body part of the subject in the magnetic field;
transmitting an RF signal in a transmit stage to be applied to the subject to be imaged such that the subject generates an MR signal in response to the magnetic field and the RF signal applied; acquiring the MR signal in a receive stage using an RF coil;
and processing the MR signal for generating an image;
wherein the RF coil is a part of an article attached to the body part of the subject so as to be carried into the magnetic field with the body part.

Preferably the RF coil is an integral part of the article.

Where the article is of a nature such as a cast or brace which remains attached to the body part of the subject, it can remain attached after leaving the imaging system and on return for a further image. In this way the patient is ready for imaging immediately without the necessity for setting up the RF coil.

Where the subject is placed on a support table for entry into the magnetic field, preferably the article is applied to the body of the subject prior to placing on the support table. That is the article may be attached onto the patient as an article of clothing such as a gown or other clothing item to be worn before the patient enters the imaging area, thus reducing the time necessary for set up in the imaging area itself. The article can be a specially constructed gown, a vest, a brassiere, a corset, a cervical neck collar, a boot, a wrapping or stretch covering for a limb formed of an elastic material such as spandex, or other article applied to the body. This can be removed when the patient departs the imaging area or in some cases may remain in place until the patient returns for further imaging.

Preferably the article is a garment worn by the subject and the RF coil is embedded in the structure of the garment.

Preferably the RF coil is free from a wired cable carrying the MR signal to the signal processing system. This arrangement can be of the type described and claimed in Published US application 2013/0221966 of the present applicant. However it is not necessary for this induction system to be used and the RF coil can be of the conventional type with includes pre-amplifiers attached to the coil and cables which carry the signals to the processing system. In this case, the electronic components required for the RF coil can also be part of the support or article with terminals for connection to a cable or the electronic components can form part of an electronic system separate from the coil itself and attached onto the coil on the article or support Preferably the signal processing system includes a plurality of channels for individual processing of separate MR signals and wherein there is provided an arrangement for generating the separate MR signals for the separate channels from the signal induced onto the RF coil. In this case, the RF coil comprises a plurality of separate loops each providing a signal to a respective one of the channels. However the same arrangement can be used with more simple coil and processing arrangements.

Preferably the RF coil comprises a volume coil configured to at least partly surround the body part of the subject so as to receive the MR signal.

Preferably the system uses the induction coil system described and claimed in the application defined above where there is provided at least one receive coil having at least one signal communication cable connected to the signal processing system for transferring the MR signal therein to the signal processing system; said at least one receive coil and said RF coil being individually tuned to a common resonant frequency for receiving said MR signal; all coil loops of said RF coil and said at least one receive coil which act only in the receive stage and do not transmit the applied RF pulse in the transmit stage having therein an arrangement to halt current flow therein at the resonant frequency during the transmit stage so as to prevent the presence of said all coil loops from interfering with the RF pulse during the transmit stage; said RF coil being arranged to communicate the MR signal therein to the signal processing system through said at least one receive coil by inducing the MR signal onto said at least one receive coil;

In this arrangement the receive coil, typically the built in body coil is located at a spacing from the RF coil such that the signal from said volume coil is induced onto said at least one receive coil at an efficiency of induction sufficient that the MR signal on said at least one receive coil is greater than the MR signal which would be generated in the absence of said volume coil; and mutual inductance between said volume coil and said at least one receive coil is insufficient to change the tuned common resonant frequency of the volume coil and the receive coil sufficiently to reduce the MR signal at said at least one receive coil to a value which is less than the MR signal which would be generated in the absence of said volume coil.

Thus preferably RF coil is free from a wired cable carrying the MR signal to the signal processing system.

Typically the built in body coil or other receive coil connects to the signal processing system which includes a plurality of channels for individual processing of separate MR signals and wherein there is provided an arrangement for generating the separate MR signals for the separate channels from the signal induced onto the RF coil. In this arrangement, the RF coil comprises a plurality of separate loops each providing a signal to a respective one of the channels.

The novel feature of described herein is the integration of a RF coil within a patient positioning or support device or an article carried by the patient such as an article of clothing or other device. The RF coils are mechanically and electrically integrated with the positioning device, allowing for close proximity of the coil to the anatomy. The integrated coil has two attributes. First, in order to conform closely to the anatomy during the formation of the support member, the circuitry must be flexible.

The preferred embodiment is a wireless RF coil, which allows for the elimination of the long cable typically used to connect the coil to the MRI scanner.

The advantages of the arrangement described herein include:
a) Eliminates the need to place and remove RF coils before, during, and after the imaging.
b) The RF coils are in close proximity to the imaging volume, significantly improving the SMR and the image uniformity.
c) The RF coils may be sterile and disposable, thereby improving reliability, eliminating the potential of cross-contamination between patients, and eliminating the burden of draping/cleaning the coils.
d) In some embodiments the RF coils are radiolucent, allowing for fluoroscopy, angiography, CT imaging, or radiation therapy to take place with a patient who will also be scanned with intraoperative MRI.
e) When used with the inductive coil arrangement, there is no need to connect the RF coils to the MRI scanner, improving imaging workflow.
f) The embedded coil configuration is conformable to the anatomy The RF coil is helpful for use with a surgical robot, where the need to maximize workspace
around the patient is essential.

Alternative embodiments include:
a) Embedded Phased array coils with detached/attached cables.
b) Embedded volumetric Transmit/Receive coils with attached detached cables.
c) Radiolucent coils as descried and illustrated in Published US applications 2011/0050226 and 2012/00286786 of the present applicants.
d) Radiolucent or non-radiolucent head fixation devices as descried and illustrated in Published US application 2013/0190604 of the present applicants.
e) Flexible or rigid embedded coil technology.

A number of different manufacturing processes can be used to produce this invention as follows:
a) Insert molding of the coil circuitry directly into the foam structure of the patient positioning device.
b) Encapsulating the coil circuitry in a rigid component upon which the foam structure is over-molded or to which the foam structure is adhered with adhesive.
c) Extruding the foam structure with a strip of coil circuitry which is fed into the extruded foam. This extrusion would then be cut into lengths for each individual foam structure.

The present arrangement provides a wireless phased array breast
coil design both for diagnostic and interventional MRI imaging that is adaptable to any MR system with the same field strength. Because of the possibility of wireless technology, the absence of the cables and active components eliminate the bulkiness on the coils and make the coil very light, flexible and patient friendly. In addition the absence of the cables and traps allows for the coils to be worn by the patient similar to a traditional brassiere accommodating to different patient sizes and being imaged on a supine position. The arrangement can provide a similar imaging performance when compared with the standard wired phased array coil. In addition, the open concept design of the elements on the wireless breast phased array coil can accommodate biopsy procedures while the lack of active components allow for the wireless coil to be sterile and/or disposable. For a wireless coil design, the design can consists of a design having two geometrically decoupled loop and butterfly coils while the isolation between the right and left elements on the proposed design is achieved either using capacitive inductive decoupling between elements or utilizing a copper shield. The elements can be wrapped around a cone shaped plastic funnel to create a conformal shape for the cups of the brassiere.

The arrangement herein also provides a wireless 9-channel phased array coil as a torso/abdomen vest coil that could be worn by the patient before entering the MRI scanner, saving setup time between patients on the scanner without sacrificing image quality performance. The coil can be a wireless inductively-coupled design that is lightweight, with minimal electronic components. Since no cable, cable baluns or active components are present, the proposed wireless coil design maximizes the available bore space for patient imaging.

Comparison between the wireless multichannel torso/abdomen vest coil with a similar in coverage traditional cabled body array coil achieve similar image quality on liver and spine imaging. Furthermore, the wireless vest coil is suitable for intraoperative imaging, such as liver ablation surgery, due to the ease of access to the patient on either side of the torso and through the coil openings, as well as for spine interventions while the patient is in the prone position. By reducing the number of components in the elements (removing preamps and interconnecting cables), the coil could be used for multi-modal imaging without impacting x-ray, such as in a CT and MR hybrid systems

The coils are designed to be arranged onto the vest style holder with side straps which enable access to the sides for interventional access.

Loading is important in the wireless coil design, and 2 cm foam spacers are integrated in the vest to provide optimum loading.

Preferably the volume coil includes a plurality of loops and each loop includes a passive decoupling circuit to halt the current in the loop during transmit stage and automatically activated during receive stage. This is called passive decoupling, which does not need a control signal and can be switched on and off automatically by body coil. When the body coil transmits, the volume coil is off, and when the body coil receives the volume coil is on.

Preferably the signal processing system includes a plurality of channels for individual processing of separate MR signals and wherein there is provided an arrangement for generating the separate MR signals for the separate channels from the signal induced onto said at least one receive coil.

For this purpose the volume coil can include a plurality of separate first loops wherein each first loop includes an addressable switch operable remotely to halt flow of current in the first loop so that each first loop can be activated in turn, and the receive coil comprises a single second loop. There is then provided a signal dividing system arranged to receive the signal from the single second loop and to calculate the separate MR signals for the separate channels from the signal induced onto the single second loop.

Alternatively the receive coil comprises a plurality of separate loops each providing a signal to a respective one of the channels.

Preferably the arrangement to halt current flow in the loops comprises an arrangement to temporarily de-tune the loop from the resonant frequency.

The term "loop" herein is used for one component or element of a complex receive coil arrangement and this term is not intended to limit the shape or structure of the individual elements defined by this term. Typically each loop is a single loop with a conductive wire or other conductive material so that current flows around the loop in response to the signal. Different materials can be used for the conductive material and certainly the terms used herein are not limited to specific materials which can be used.

For example such a "loop" can be formed by a complex volume coil which surrounds a part to be imaged.

The intention in the above arrangement is that said the first coil is free from a wired cable carrying the MR signal to the signal processing system. This can provide a number of significant advantages.

The arrangement provided herein therefore consists of a cable-less volume coil, which works by coupling with the built in body coil of the MR magnet. This volume coil does not have as many components as a conventional MR imaging coil. The design can be defined by a birdcage resonator and is used as a volumetric coil.

This arrangement can provide one or more of the following features and advantages:
a) Inductive volume coils can achieve equal or better images compared with commercial phased array volume coils. The coils herein can provide highly uniform images with good SNR numbers;
b) there is no limitation to the number of channel regardless of the number of receivers in the system.
c) No cables with external cable traps are required to connect the coil to the system.
d) It is significantly easier to build as the coil contains only passive elements.
e) There is no need for internal baluns, preamps, connection cables, cable traps, or external connector blocks, or extension cables.
f) The coil has smaller physical dimensions (size, weight) compared with similar (same field of view) phased array volume coil.
g) the cable-less volume coil can improved hospital workflow.
h) patient positioning and surgical access is significantly improved.
j) The possibility of patient burns resulting from patient skin-to-coil cable contact is completely eliminated.
k) Increased patient safety.
l) Passive decoupling is provided for eliminating crosstalk between the inductive wireless coils to the built-in body coil during the transmit phase. Therefore, B1 distortion, coil heat and image non uniformity caused by B1 distortion is eliminated. B1 is RF field generated by the built in body coil.

Both 1.5T and 3T coil imaging is comparable to the existing commercial phased array Head Coil and provide very good image uniformity and high SNR.

A number of possible arrangements can be used within this broad definition.

Firstly the second coil can be a built in body coil carried on the magnet. Such body coils are typically available on magnet systems.

The second coil or body coil can act as the transmit coil or another dedicated coil can be separately used.

There can be only two coils using the inductive coupling to transfer the signal to the processor or there may be a stack of three coils or even more.

In this arrangement, the first coil can be located inside the body of a patient and the second coil is arranged outside the body of the patient. Typically in this arrangement, the second coil is as close as possible to the exterior of the patient and this in turns communicates inductively to the body coil (or other coil) around the patient.

The first coil is arranged to be located as close as physically possible to the subject and the second coil is arranged to be located at a position spaced from the subject greater than that of the first coil so as to receive the signal inductively and transfer it to the processing unit.

The arrangement herein is predicated on the discovery that providing a first coil as close as possible to the part to be imaged and covering as small a volume as possible generates a signal which has significantly greater signal to noise ratio than a second coil located at a spacing from the part. Then the signal picked up by the first coil is communicated inductively to the second coil even though there are significant losses in so doing. It has been found that the signal from the first coil is induced onto said at least one second loop at an efficiency of induction (less than 100%) sufficient that that the MR signal on second coil is greater than the MR signal which would be generated in the absence of the first coil. This includes the possibility of a catheter coil being used which increases the signal to the surface coil. That is there is a magnifying effect by providing the first coil close to the subject and then communicating the signal to the second coil despite the losses in the inductive coupling.

Another issue which arises is that mutual inductance between the coils can change the tuned common resonant frequency of the loops to reduce the MR signal unacceptably. Typically therefore it would be considered that the problems of mutual inductance changing the tuned frequency would at least balance and more likely outweigh the advantages obtained by providing the additional first coil. However this has been found not to be so. Provided the distances are carefully managed by experiments to determine what distances provide an advantage without adversely affecting the tuning to a situation where the MR signal is at a value which is less than the MR signal which would be generated in the absence of said at least one first loop, significant advantages can be obtained.

One issue which arises and is addressed herein is that of how to generate separate signals for separate channels of the signal processing unit in order to take advantage of the high speed imaging which can be obtained by using parallel channels such as by SENSE or SMASH or other more recent techniques. Preferably each loop includes an addressable switch operable remotely to halt flow of current in the loop so that each loop can be activated in turn.

In a first embodiment to overcome this difficulty, the first coil includes a plurality of separate loops and there is provided an arrangement for generating the separate MR signal for the separate channels from the signal induced onto the second coil.

In one arrangement, each first loop includes an addressable switch operable remotely to halt flow of current in the first loop. In this way each first loop can be activated in turn. In this arrangement using conventional MRI equipment where the body coil has a single output. In this arrangement, the individual element sensitivity profiles can be obtained to perform parallel imaging. A signal processing system is arranged to receive the signal from the single channel, and along with the sensitivity profiles will separate the combined single channel into its individual elements for processing by the scanner. The individual signals from coils can be determined by measuring what are known as the Sensitivity Profile and Noise Correlation Matrix of the coil using those factors to determine the individual signals for the separate channels. In this arrangement, the sensitivity profile and possibly Noise Correlation Matrix of the single second coil can be determined by operating the switch to turn off each of the first coils. After this is determined, the sensitivity profile and Noise Correlation Matrix of each of the first loops can be determined by activating only each one in turn with the others turned off and then by subtracting the signal obtained from single second coil from the total signal obtained by the second coil and the activated one of the first loops. The Sensitivity Profile and possibly the Noise Correlation Matrix are then used to determine from the single output of the single second receive coil the required individual signals required for the separate channels of the processing system. For the parallel imaging, a base image is obtained with RF body coil only. Utilizing the switching of the individual loops, an image for each of the inductive loops is obtained in succession as well as any possible combination of them. Thus, by a subtraction of images from the body coil base image, a picture of the sensitivity fields and correlation matrices between coils is obtained. Once this arrangement is obtained an under sampling during the parallel imaging can be unfolded. This technique can be extended in space and time domain as well with methods like GRAPPA. In a second arrangement applicable to arrangements with a body coil which has separate loops connected to separate channels, the arrangement of the body coil has been found to provide the required signal to each respective one of the channels.

In accordance with another important aspect of the invention, the coil is provided with a switch which acts to deactivate the coil after a period of time. Thus the switch can be moved to open circuit when a time period after first activation has elapsed. In this way, the active life of the coil can be controlled. This can be limited for example to a number of hours so that the coil is a one time use product. Thus the switch is activated on receipt of the first RF pulse and then has a timing circuit which times out to operate the switch to open circuit preventing further ruse of the coil assembly. In another arrangement, the switch may act in response to sterilization so that it allows a certain number of sterilizing actions before moving to open circuit. In yet another arrangement, the total allowable lifetime of the coil can be predetermined by the manufacturer and then actively enforced against users who may try to use the product beyond its life. This arrangement allows the coil to be a one time use product requiring it to be discarded after the one time use with this protocol being fully enforced against users wanting to ignore it.

In order to make the product disposable, components can be provided to control the operation of the loops which avoids the use of higher cost components such as transistors and variable elements. This can be achieved by using de-tuning of the coil to switch the coil when it is not required to respond to the RF signal. Thus de-tuning of the coil to a resonant frequency sufficiently different from the RF frequency is equivalent or achieves the same result as switching the loop to open circuit. This can be achieved in many ways and in particular by moving of a cooperating coil to a position close to the coil to change the tuning.

In order to ensure the separate loops are de-coupled so as to avoid interfering with the resonant tuning, conventional de-coupling techniques can be used including geometric arrangements of the loops, capacitive de-coupling, inductive decoupling and the use of a separate additional loop which acts to inductively couple between two of the separate loops to provide the necessary current cancelling actions necessary to provide the de-coupling between the two separate loops.

All of these techniques are known to persons skilled in the art.

The coil size (with built in preamplifiers) and cable are the primary issues that affect coil performance, workflow, sterilization and safety. This new design described herein can greatly improve coil performance, workflow, sterilization and safety, since it does not include any of these components.

In the arrangement where the first coil is a phased array including a plurality of separate loops, one or more loops of the phased array coil are without preamplifiers and no cables, no physical connection to the scanner, thus providing a so called "wireless coil". These wireless coil elements are resonators and tuned at MR scanner working frequency. These wireless coil elements or loops are decoupled from each other using conventional techniques by coil loop overlap, capacitive techniques including shared conductor, inductive and geometry (such as quadrature) methods. These wireless coil elements can be transverse electromagnetic (TEM) coil and receive only coils with good decoupling between coil elements by using current technology without cable and preamplifier.

These wireless coil elements are inductively coupled in the receive stage to the built in RF body coil. In a multiple system using additional coils, these wireless coils can couple with each other in a successive manner to larger and/or smaller coils that consequently couple to the built in RF body coil. These coils are passively detuned from the Transmit portion of the TX/RX Whole

Body RF coil or other transmit coil during the transmit stage.

The frequency of operation covers the entire spectrum of RF. The wireless coil elements combination can be inductively coupled multi loops along the magnet axis or off axis.

The coil elements are passively decoupled from transmit coil during the transmit stage. The transmit coil can be the built in body coil in the MR scanner or can be a local transmit coil or transmit phased array. Or a transceiver coil can work with a multi transmitter system. The wireless coil elements size can be as large as head or body coil and as small as intra-cardiac coil (diameter <10mm).

The sensitivity of the wireless coil elements can be adjusted by de-tune, insert impedance and other methods to eliminate coil crosstalk and optimize signal to noise ratio.

The distance between wireless coil elements and pickup coils can be adjusted for optimized SNR bearing in mind the competing requirements of reducing mutual inductance to prevent de-tuning and maximizing signal transfer efficiency.

The distance between wireless coil elements and subject to be imaged can be adjusted for optimized SNR bearing in mind the competing requirements of reducing load and keep the Q factor higher of each coil elements, so that each coil element can get the maximum MR signal from the subject to be imaged.

### BRIEF DESCRIPTION OF THE DRAWINGS

One embodiment of the invention will now be described in conjunction with the accompanying drawings in which:
Figure 1 is a schematic illustration of an MRI system including a first embodiment of the present invention where the coil is incorporated into a head support.
Figure 2 is a schematic illustration of the head support of Figure 1.
Figure 3 is an isometric view of the head support of Figure 2.
Figure 4 is an isometric view of the head support of Figure 2 showing an alternative arrangement of the coil.
Figure 5 is an isometric view of the head support of Figure 2 showing a further alternative arrangement of the coil.
Figure 6 is an isometric view of the head support of Figure 2 showing a yet further alternative arrangement of the coil.
Figures 7A and 7B are front and rear schematic illustrations of the coil incorporated into a garment to be worn by the patient.
Figure 8 is a schematic illustration of the coil incorporated into a bolster for support of the pelvis.
Figure 9 is a schematic illustration of the coil incorporated into a knee brace to be worn by the patient.
Figure 10A is a schematic illustration of the coil incorporated into a leg cast to be worn by the patient.
Figure 10B is a schematic illustration of the coil incorporated into a foot brace/cast to be worn by the patient.
Figure 11 is a schematic illustration of the coil incorporated into a cervical spine collar to be worn by the patient.
Figure 12 is a schematic illustration of the coil incorporated into a prone head pillow for supporting the patient.
Figure 13 is a schematic illustration of the coil incorporated into a vacuum bean bag support for supporting the patient.
Figure 14 is a schematic illustration of the coil incorporated into a brassiere to be worn by the patient.
Figure 15 is a cross section through the support of Figure 2 at a resilient portion of the support where the coil is embedded in the resilient material.
Figure 16 is a cross section through the support of Figure 2 showing the coil embedded in an extruded strip.
Figure 17 is a cross section through the support of Figure 2 at a rigid portion of the support where the coil is embedded in a resilient material over-molded onto a rigid frame member of the support.

In the drawings like characters of reference indicate corresponding parts in the different figures.

### DETAILED DESCRIPTION

The apparatus for MR imaging of a subject includes a conventional cylindrical MR magnet 10 operable by a field control system to generate a variable magnetic field to be applied to the subject.

The MR system includes an RF transmit arrangement 12 for generating RF pulses in a transmit stage to be applied to the subject to be imaged and a receive arrangement for acquiring the MR signal in a receive stage with a signal processing system 13 for receiving the MR signal for carrying out signal processing by which an image is generated. As is well known, the subject generates an MR signal in response to the magnetic field and the RF signal applied which is detected and processed to generate an image. The arrangement is well known and a suitable system is available from Siemens.

Typically the magnet 10 carries an RF coil known as a body coil 14 which is mounted on the cylindrical magnet housing so as to surround the patient. This is usually used as the transmit coil. However separate transmit can be used. The body coil can also operate as the receive coil. However again separate receive coils can be used. The transmit and receive coils can be the same coils or can be provided by separate coils.

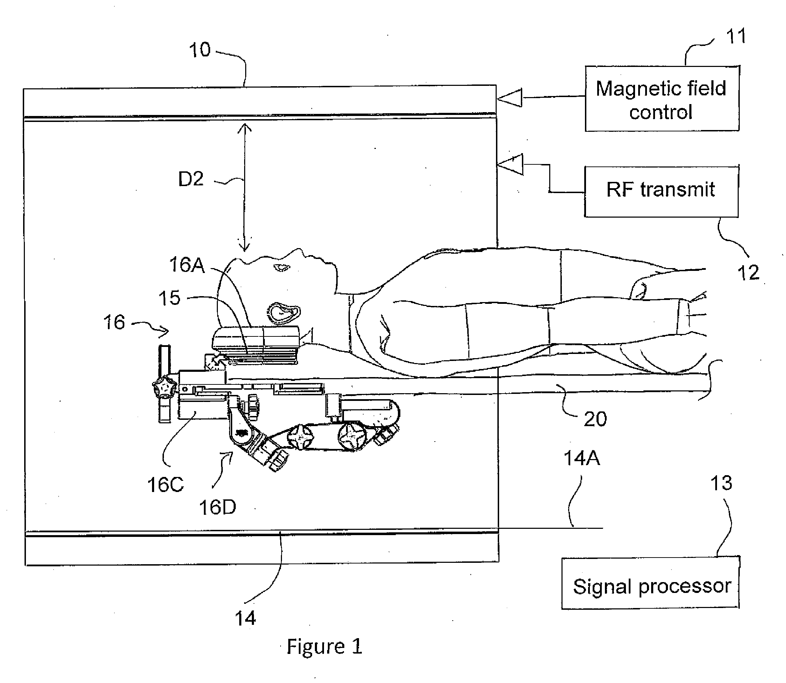

In the first embodiment shown in Figure 1, the transmit coil is defined by the body coil 14. The receive coil arrangement comprises coil loop 15 located on a support 16 for a part to be imaged of the body of the patient. The receive coil arrangement further comprises an outer coil defined by the body coil 14 surrounding the coil 15. The second outer coil 14 has a signal communication cable 14A connected to the signal processing system 13 for transferring the MR signal therein to the signal processing system.

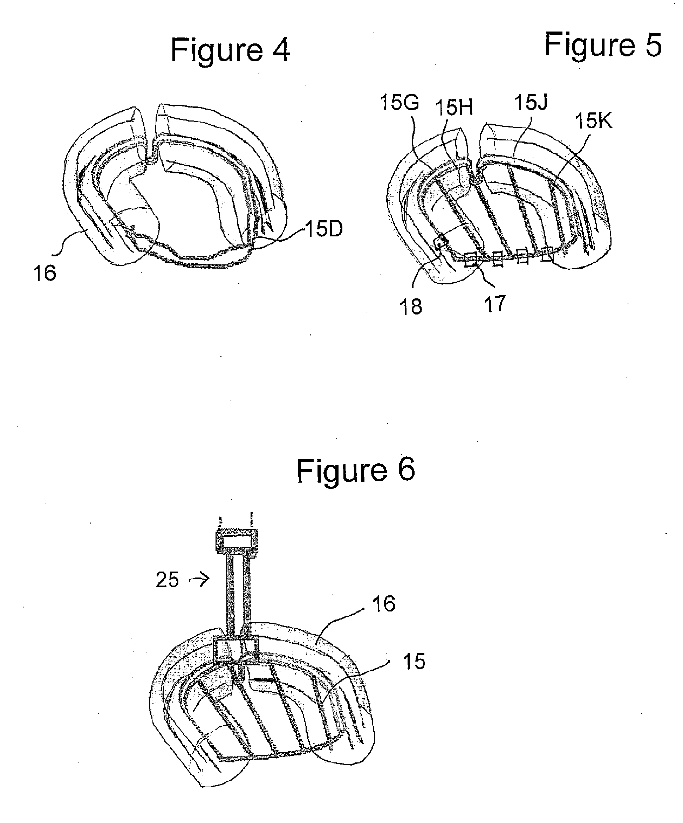

The coil 15 can be formed of a single loop 15C as shown in Figure 4 where the loop defines a conventional butterfly with an overlap at the center 15B where the coil doubles back at the ends 15C.

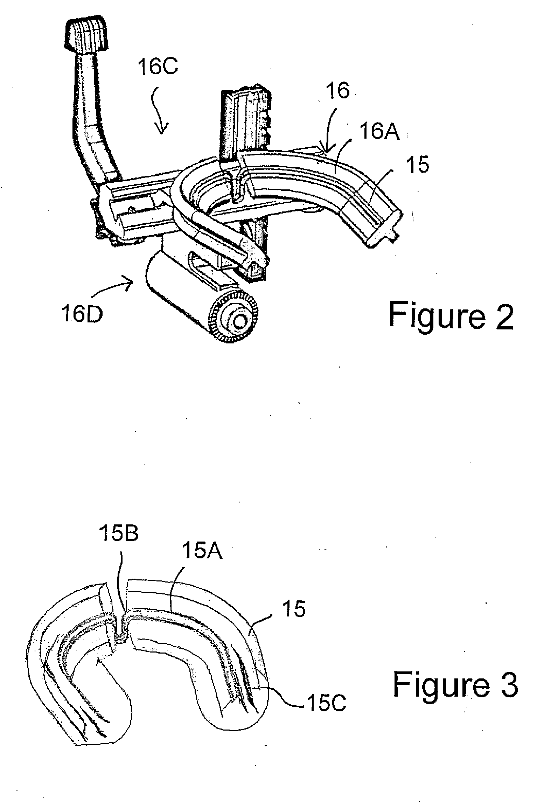

The coil 15 can be formed of a single loop 15A as shown in Figure 3 where the loop defines a conventional butterfly with an overlap at the center 15B where the coil has end sections 15D which connect at the front of the support 16.

In Figure 5, the coil 15 is formed by a phased array of loops 15G, 15H, 15J, 15K.

All of the loops of the coils 15 and the body coil 14 are individually tuned by a tuning component such as capacitors schematically indicated at 17 to a common resonant frequency for receiving said MR signal using conventional tuning devices well known to a person skilled in the art.

All of the coil loops of the coils 15 which act only in the receive stage and do not transmit the applied RF pulses in the transmit stage have therein an arrangement schematically indicated at 18, such as a passive block circuit with capacitors, inductor and pin diodes, to halt current flow therein during the transmit stage so as to prevent the presence of said all coil loops from interfering with the RF pulses during the transmit stage. Devices of this type are known so that explanation of the operation is not necessary.

The loop of the innermost coil 15 is arranged to communicate the MR signal therein to the signal processing system through the outer coil 14 by inducing the MR signal onto the coil 14.

The intention in the above arrangement is that said the coils 15 are free from a wired cable carrying the MR signal to the signal processing system.

Typically in this arrangement, the coil 15 is as close as possible to the exterior of the patient and this communicates inductively to the body coil (or other coil) around the patient.

Thus the coil 15 is arranged to be located as close as physically possible to the subject and the second coil or body coil 14 is located at a position spaced from the subject greater than the that of the coil 15 so as to receive the signal inductively and transfer it to the processing unit.

The coil 15 is as close as possible to the part to be imaged and covers or surrounds as small a volume as possible so as to receive noise from as small a volume as possible and so as to receive as much signal as possible, bearing in mind that the signal falls rapidly as is passes through the tissue. This therefore generates a signal which has significantly greater signal to noise ratio than a second coil located at a greater spacing from the part. Then the signal picked up by the coil 15 is communicated inductively to the coil 14 even though there are significant losses in the inductive communication. The signal from the coil 15 is induced onto the coil 14 at an efficiency of induction (less than 100%) but sufficient that that the MR signal on coil 15 is greater than the MR signal which would be generated on coil 14 in the absence of the coil 15. That is there is a magnifying effect by providing the coil 15 close to the subject and then communicating the signal to the coil 14 despite the losses in the inductive coupling.

It will be appreciated that the coil 14 also receives signals directly from the part being imaged which signals are added to the signals communicated inductively. However in each case, the inductively coupled signal is much greater than the directly detected signal.

Another issue which arises is that mutual inductance between the coils 14 and 15 can change the tuned common resonant frequency of the loops to reduce the MR signal unacceptably. Thus the spacing between them must be sufficient such that the amount of mutual inductance does not change the tuning frequency sufficiently to interfere with the tuning to a level where the acquisition of the signal is degraded. This is of course a trade off and the actual distance spacing between the particular coils of a specific embodiment must be determined by simple experimentation to move the coils to the required position to obtain the best signal having the best signal to noise ratio.

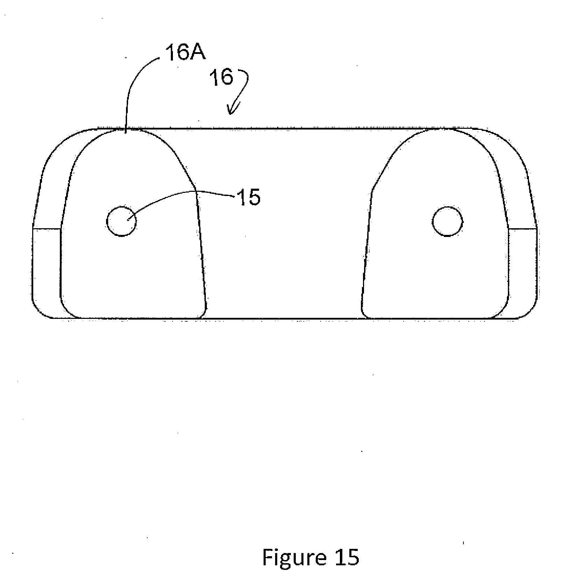

As shown in Figures 2 to 6, 8 and 15, the RF coil is embedded within the supporting element by being cast within the resilient material such as foam, silicone or other material during the formation of the supporting element. In this embodiment the support is a conventional horse-shoe support with an upper surface 16A extending around the back of the head an on which he head rests. The front is open to allow the neck to pass. The support is shaped to provide the required stiffness to support the weight of the head while allowing the upper surface to flex for comfort.

The horse-shoe support is carried on a conventional frame 16C attached to a patient support table 20 by an adjustment system 16D so that the subject or patient is carried on the support table and the supporting element is removable from the table and carries the RF coil 15 as an integral element therewith.

As set forth above, the RF coil 15 is integrated with the support 16 element by insert molding of the loop and the coil circuitry 17, 18 thereof directly into a resilient material forming the structure of the support element

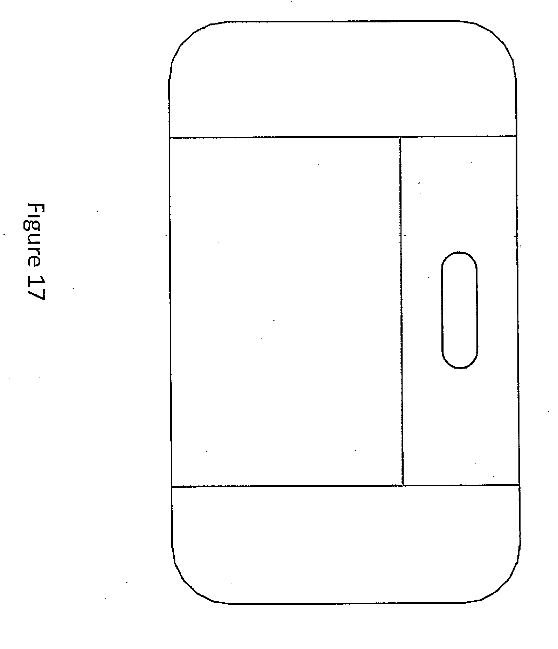

As shown in Figure 17, as an alternative, the RF coil is integrated with the support element 16G by encapsulating the loop and coil circuitry thereof onto a rigid component 21 upon which a resilient material 22 is over-molded or adhered to with adhesive with the coil loop in the resilient material.

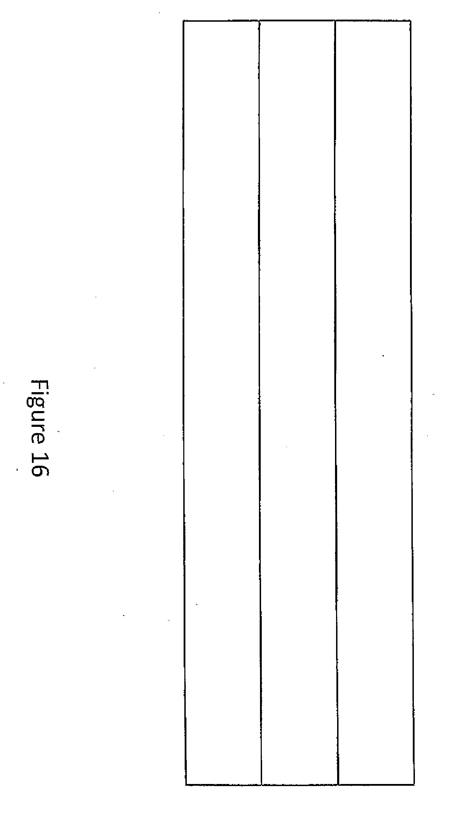

As shown in Figure 16, the RF coil 15K is integrated with the support element 16 by extruding a resilient material 16K with a strip of coil circuitry 15K thereof which is fed into the extruded resilient material and thereafter the extruded material is cut into lengths.

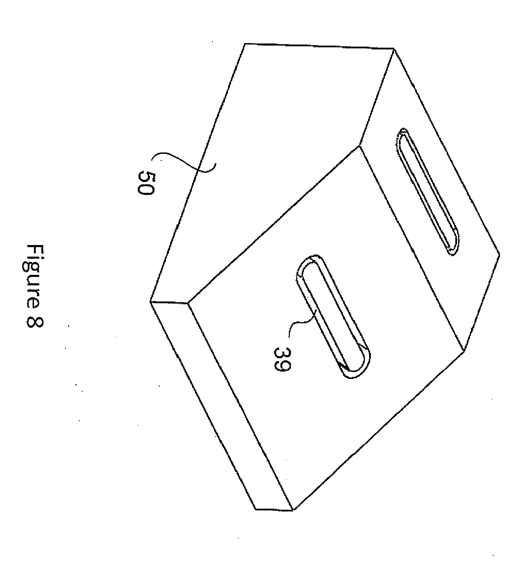

In Figure 8 the coil 39 is incorporated into a bolster 50 of a conventional construction for support of the pelvis.

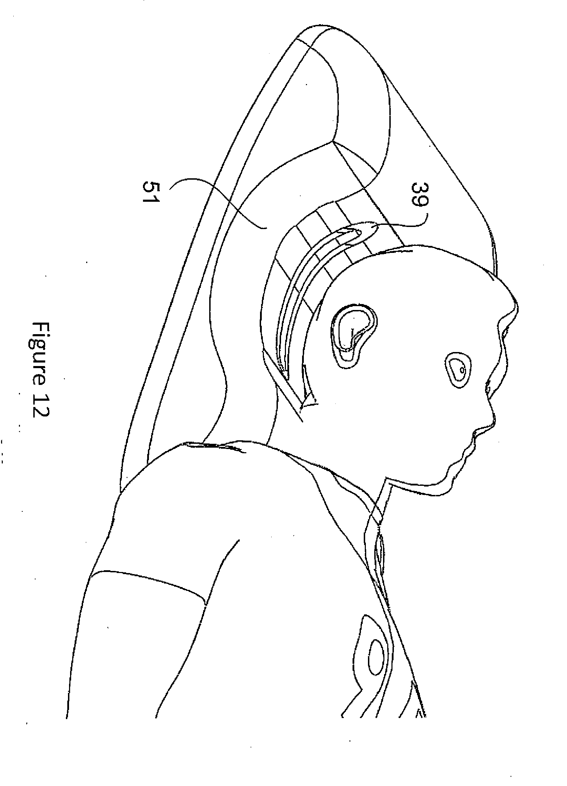

In Figure 12 the coil 39 is incorporated into a prone head pillow 51 of a conventional construction for supporting the head of the patient.

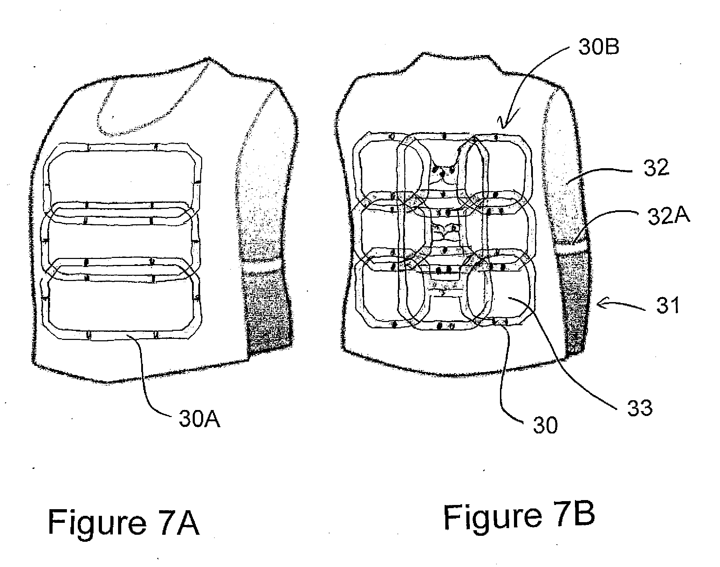

As shown in Figures 7, 9, 10A, 10B, 11, 13 and 14 the RF coil is a part of an article attached to the body part of the subject so as to be carried into the magnetic field with the body part. That is the coil is an integral part of the article where the article is a vest as described in more detail hereinafter, a knee brace, a cast, a cervical spine collar and a brassiere.

In each case the article can remain attached to the body part of the subject after leaving the imaging system and on return for a further image.

In each case, the subject is placed on a support table for entry into the magnetic field of a magnet where the article is applied to the body of the subject prior to placing on the support table.

In each case, the article is a garment or other element worn by the subject and the RF coil is embedded in the structure of the garment.

In all cases except Figure 6, the RF coil is preferably free from a wired cable carrying the MR signal to the signal processing system. However in each case a wired connection can be used for example as shown in Figure 6 where a wire connection 25 of a conventional nature is used. In particular starting from Figures 7A and 7B, a multi coil article 30 is embedded inside the a thoracic device 31, that can be a medical gown appropriate for Magnetic resonance Imaging procedures as well as thoracic casting and braces that made from a set of materials compatible with the requirements of Magnetic resonance Imaging. Furthermore, the coils, array of coils can be attached or embedded inside the casting or thoracic restraining devices either being part of the mold and being molded over or extruded in pieces that later can be mechanically or electrically connected together.

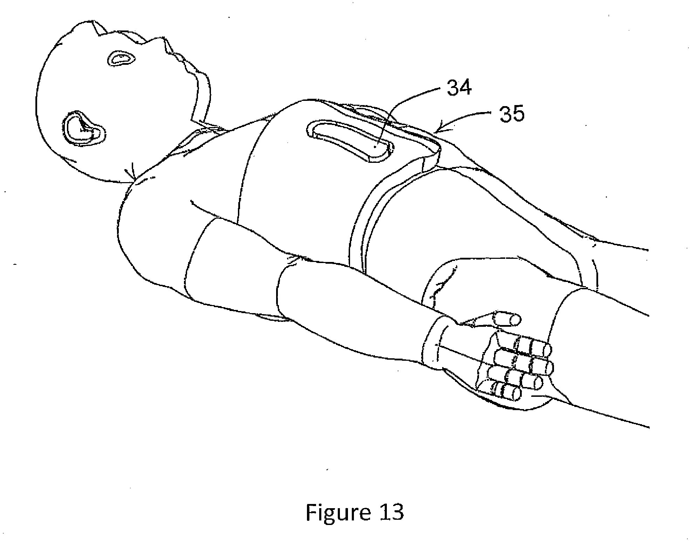

Thus the arrangement of Figures 7A and 7B also provides a wireless 9-channel phased array coil 30 as a torso/abdomen vest coil that can be worn by the patient before entering the MRI scanner. The coil can be a wireless inductively-coupled design that is lightweight, with minimal electronic components. No cable, cable baluns or active components are present. The wireless vest coil is suitable for intraoperative imaging, such as liver ablation surgery, due to the ease of access to the patient on either side of the torso at openings 32 and through the coil openings 33. Besides the thoracic applications, similar coil technology and applications can be considered for the pelvic area where multi coil articles in term of a phased array or volumetric design 34 can be strategically wrapped around the pelvic casting 35 as shown in Figure 13 to optimize the coil's effectiveness over the pelvic area.

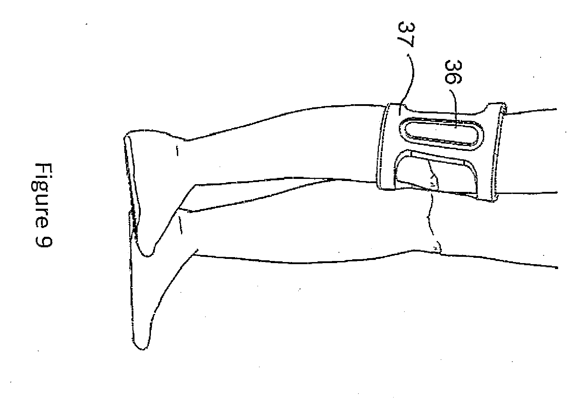

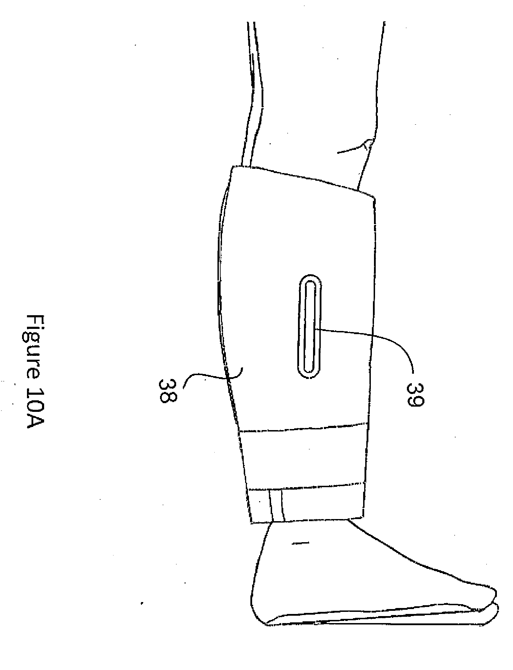

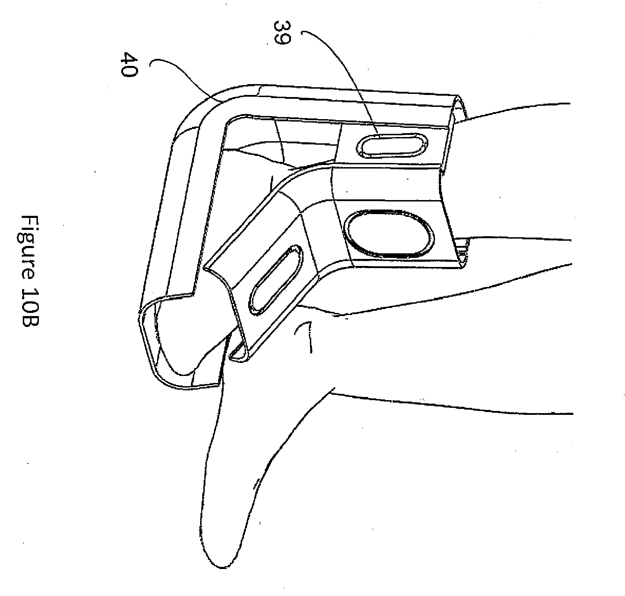

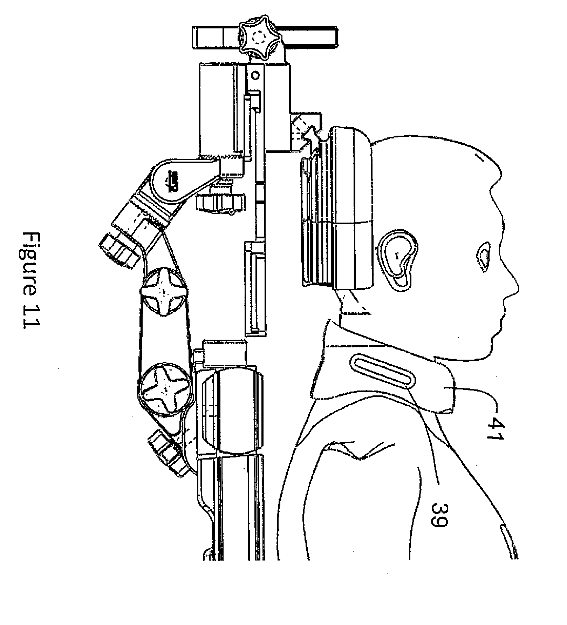

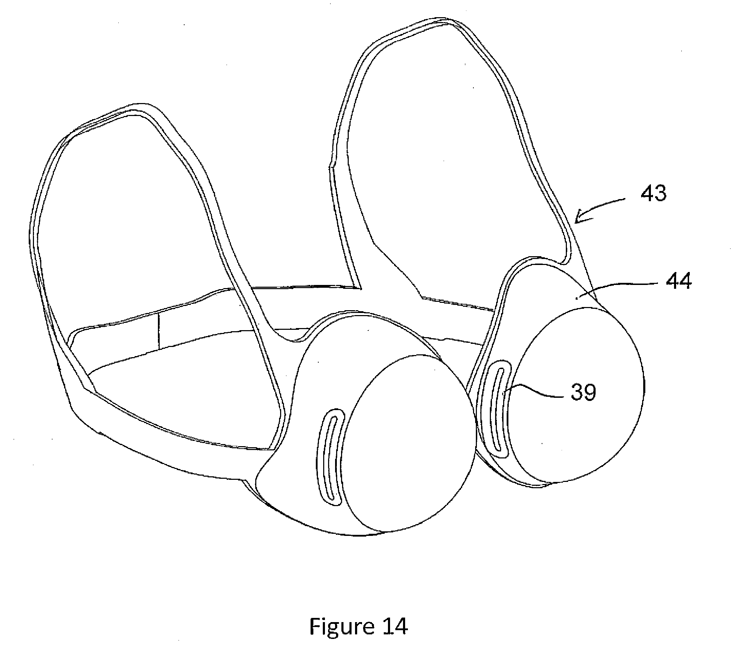

Similar designs can be envisioned for the knee brace 37 of Figure 9 where the proximity of the coils 36 to the examined structure is vital and coil articles have to be as close as possible to the region of Interest. The coils 39 can be part of the leg cast 38 of Figure 10A, the foot or ankle cast 40 of Figure 10B, the cervical neck collar 41 of Figure 11, the vacuum bean bag 35 at the torso of figure 13, braces or brassiere 43 of Figure 14.

Thus in Figure 14 is shown the RF coil 39 incorporated into a brassiere 43 to be worn by the patient for imaging of and procedure on the breast.

For a wireless coil design, the design can consists of a design having two geometrically decoupled loop and butterfly coils while the isolation between the right and left elements on the proposed design is achieved either using capacitive inductive decoupling between elements or utilizing a copper shield. The elements can be wrapped around a cone shaped plastic funnel 44 to create a conformal shape for the cups of the brassiere.

One of the key elements in MRI image quality is the use of the appropriate RF coil. Specifically for breast imaging applications, RF coils have to be designed such that provide the optimum SNR and uniformity for unilateral and bilateral imaging, as well as on the peripheral lymph node images, as well as to increase patient comfort, introducing structures that ensure patient comfort for patients on a prone position. Traditionally the trend towards a large element count for RF coils providing higher image quality that can assist in better detection and identification of tumors and lesions within the soft tissue. However the introduction of clustered multichannel arrays restricts the comfort of the patient and inhibits the ability of a physician to perform interventional procedures on the breast with the same coil. That is the reason that there are in general two sets of coils, one for the diagnostic imaging and a second set for biopsy applications. Furthermore traditional designs of RF coils include active components and cables with cable traps to prevent heating. This leads to bulky, heavy weight and complicated coil design with increased patient setup time and imaging restriction due to patient size.

The present arrangement provides a wireless phased array breast coil design both for diagnostic and interventional MRI imaging that is adaptable to any MR system with the same field strength. Because of the wireless technology, the absence of the cables and active components eliminate the bulkiness on the coils and make the coil very light, flexible and patient friendly. In addition the absence of the cables and traps allows for the coils to be worn by the patient similar to a traditional bra accommodating to different patient sizes and being imaged on a supine position. The arrangement can provide a similar imaging performance when compared with the standard wired phased array coil. In addition, the open concept design of the elements on the wireless breast phased array coil can accommodate biopsy procedures while the lack of active components allow for the wireless coil to be sterile and/or disposable.

In Figure 7 is shown the RF coil incorporated into a vest to be worn by the patient for imaging of and procedure on the torso.

Multichannel phased array coils are the dominant technology for MRI imaging of the abdomen, torso and pelvis. Over the past few years, massive multichannel arrays for abdominal imaging applications utilizing parallel imaging and coverage in order to acquire an artifact free motion free MRI Image Although, these massive arrays were trying to address the image quality issue for abdomen and torso, they pose significant disadvantages in terms weight of the coil on the patient as well as the presence of cables, and cable traps that are a near proximity to the patient. Specifically, the need for cable traps and insulation on cables is to reduce risk of patient burning due to RF heating caused by large induced eddy currents in the copper or by faulty cable traps. Furthermore, the need to have a minimum spacing between the cable traps and the patient and due to the size of cable traps and the peripheral active electronics restrict significantly the free space available for patient imaging inside the magnet bore. In addition massive RF coil arrays with large number of cables present further disadvantages for interventional MRI applications where they interfere with or impeded the surgical workflow.

The arrangement herein provides a wireless 9-channel phased array coil as a torso/abdomen vest coil that could be worn by the patient before entering the MRI scanner, saving setup time between patients on the scanner without sacrificing image quality performance. The coil is a wireless inductively-coupled design that is lightweight, with minimal electronic components.

Since no cable, cable baluns or active components are present, the proposed wireless coil design maximizes the available bore space for patient imaging. Comparison between the wireless multichannel torso/abdomen vest coil with a similar in coverage traditional cabled body array coil achieve similar image quality on liver and spine imaging. Furthermore, the wireless vest coil is suitable for intraoperative imaging, such as liver ablation surgery, due to the ease of access to the patient on either side of the torso and through the coil openings, as well as for spine interventions while the patient is in the prone position. By reducing the number of components in the elements (removing preamps and interconnecting cables), the coil could be used for multi-modal imaging without impacting x-ray, such as in a CT and MR hybrid systems The 9-element wireless torso/abdomen vest coil is shown in Figures 7A and 7B. For the wireless design the anterior part of the coil consists of 3 geometrically decoupled loops 30A while the posterior part of the coil consists of 6 geometrically decoupled loop and butterfly elements 30B. The combination of the anterior and posterior elements for the wireless coil defines a 45cm FOV for imaging. Each posterior element is tuned to either 63.6 MHz. and are passively detuned from the RF body coil during transmit mode, while incorporate an RF fuse. Due to the absence of preamplifier decoupling in the wireless vest coil design, each element is decoupled from its neighbours using geometric and capacitive means, achieving isolation greater than 15db amongst all coil elements. Since the coil is wireless, the Q factors (unloaded and loaded) of the coil are great parameters for the efficiency of the coil. The coils are designed to be arranged onto a vest style holder with side straps 32A which enable access to the sides for interventional access. Loading is important in the wireless coil design of the coils 30A and 30B, and 2 cm foam spacers (not shown) are integrated in the vest to provide optimum loading.

## Claims

1. A method for MR imaging of a subject comprising
generating a variable magnetic field to be applied to a body part to be imaged of the subject; supporting the body part of the subject in the magnetic field by engaging the body part with a supporting element;
transmitting an RF signal in a transmit stage to be applied to the subject to be imaged such that the subject generates an MR signal in response to the magnetic field and the RF signal applied; acquiring the MR signal in a receive stage using an RF coil;
and processing the MR signal for generating an image;
wherein the RF coil is an integral element with the supporting element.

2. The method according to claim 1 wherein the RF coil comprises a volume coil configured to at least partly surround the body part of the subject so as to receive the MR signal.

3. The method according to claim 1 or 2 wherein there is provided at least one receive coil having at least one signal communication cable connected to the signal processing system for transferring the MR signal therein to the signal processing system; said at least one receive coil and said RF coil being individually tuned to a common resonant frequency for receiving said MR signal; all coil loops of said RF coil and said at least one receive coil which act only in the receive stage and do not transmit the applied RF pulse in the transmit stage having therein an arrangement to halt current flow therein at the resonant frequency during the transmit stage so as to prevent the presence of said all coil loops from interfering with the RF pulse during the transmit stage; said RF coil being arranged to communicate the MR signal therein to the signal processing system through said at least one receive coil by inducing the MR signal onto said at least one receive coil;

4. The method according to any preceding claim wherein said at least one receive coil is located at a spacing from said RF coil such that:
the signal from said volume coil is induced onto said at least one receive coil at an efficiency of induction sufficient that the MR signal on said at least one receive coil is greater than the MR signal which would be generated in the absence of said volume coil; and
mutual inductance between said volume coil and said at least one receive coil is insufficient to change the tuned common resonant frequency of the volume coil and the receive coil sufficiently to reduce the MR signal at said at least one receive coil to a value which is less than the MR signal which would be generated in the absence of said volume coil.

5. The method according to any preceding claim wherein the RF coil is embedded within the supporting element.

6. The method according to any preceding claim wherein the subject is carried on a support table and the supporting element is removable from the table and carries the RF coil as an integral element therewith.

7. The method according to any preceding claim wherein the RF coil is integrated with the support element by insert molding of coil circuitry thereof directly into a resilient material forming the structure of the support element

8. The method according to any preceding claim wherein the RF coil is integrated with the support element by encapsulating a coil circuitry thereof in a rigid component upon which a resilient material is over-molded or adhered to with adhesive.

9. The method according to any preceding claim wherein the RF coil is integrated with the support element by extruding a resilient material with a strip of coil circuitry thereof which is fed into the extruded resilient material wherein the extruded material is cut into lengths.

10. A method for obtaining an MR image of a subject in an MR imaging system comprising
generating a variable magnetic field to be applied to a body part to be imaged of the subject; supporting the body part of the subject in the magnetic field;
transmitting an RF signal in a transmit stage to be applied to the subject to be imaged such that the subject generates an MR signal in response to the magnetic field and the RF signal applied; acquiring the MR signal in a receive stage using an RF coil;
and processing the MR signal for generating an image;
wherein the RF coil is a part of an article attached to the body part of the subject so as to be carried into the magnetic field with the body part.

11. The method according to claim 10 wherein the RF coil is an integral part of the article.

12. The method according to claim 10 or 11 wherein the article remains attached to the body part of the subject after leaving the imaging system and on return for a further image.

13. The method according to any one of claims 10 to 12 including placing the subject on a support table for entry into the magnetic field of a magnet wherein the article is applied to the body of the subject prior to placing on the support table.

14. The method according to any one of claims 10 to 13 wherein the article is a garment worn by the subject and the RF coil is embedded in the structure of the garment.

15. The method according to any preceding claim wherein said RF coil is free from a wired cable carrying the MR signal to the signal processing system.
